(19) Europäisches Patentamt
European Patent Office
Office européen des brevets

(11) Veröffentlichungsnummer : **0 069 063**
**B1**

(12) # EUROPÄISCHE PATENTSCHRIFT

(45) Veröffentlichungstag der Patentschrift : **22.01.86**

(51) Int. Cl.⁴ : **C 07 C 87/28**, C 07 C 93/14, C 07 C 87/54, C 07 D333/20

(21) Anmeldenummer : **82810252.5**

(22) Anmeldetag : **14.06.82**

(54) **Aminoalkyl-substituierte aromatische oder heterocyclische Verbindungen und Verfahren zu deren Herstellung.**

(30) Priorität : **19.06.81 GB 8119008**

(43) Veröffentlichungstag der Anmeldung : **05.01.83 Patentblatt 83/01**

(45) Bekanntmachung des Hinweises auf die Patenterteilung : **22.01.86 Patentblatt 86/04**

(84) Benannte Vertragsstaaten : **CH DE FR GB IT LI NL**

(56) Entgegenhaltungen :
**FR-A- 919 132**
**FR-A- 2 222 355**
**US-A- 3 830 923**
**JOURNAL OF ORGANIC CHEMISTRY, Band 31, Oktober 1966, Seiten 3093-3098, Washington D.C., USA, A.C. COPE et al.: "Friedel-crafts reactions of amino tertiary alcohols"**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Klybeckstrasse 141**
**CH-4002 Basel (CH)**

(72) Erfinder : **Howell, Frederick Harold, Dr.**
**27 High Bank**
**Atherton Lancashire M29 9HZ (GB)**

**EP 0 069 063 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue aminoalkyl-substituierte aromatische oder heterocyclische Verbindungen und ein Verfahren zu deren Herstellung.

Die US Patentschrift 2,900,369 beschreibt 1,4-Bis(6-Amino-2-methylhex-2-yl) benzol und das entsprechende Polypyromellitimid. Die französische Patentschrift 919,132 beschreibt Diamine, die durch Friedel-Crafts Alkylierung von Aromaten mit Allyl- bzw. Methallylamin hergestellt werden, und anschliessend zu linearen Polyamiden umgesetzt werden.

Gegenstand der vorliegenden Erfindung sind Verbindungen der Formel I

$$R_4(QNH_2)_2 \qquad (I)$$

worin die Reste $—QNH_2$ gleiche oder verschiedene Gruppen der Formel II

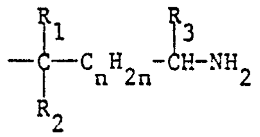
(II)

bedeuten, worin n eine ganze Zahl von 1-15, $R_1$ $C_{1-8}$-Alkyl, $R_2$ $C_{1-4}$-Alkyl oder $R_1$ und $R_2$ zusammen mit dem C-Atom, an das sie gebunden sind, eine $C_{5-8}$-Cycloalkylengruppe und $R_3$ Wasserstoff, $C_{1-6}$-Alkyl, $C_{3-8}$-Cycloalkyl oder $C_{6-10}$-Aryl bedeuten, mit der Massgabe, dass wenn $R_4$ = 1,4-Phenylen, n = 3 und $R_1 = R_2$ = Methyl sind, $R_3$ verschieden von Wasserstoff ist, und $R_4$ einen gegebenenfalls durch eine oder zwei gleiche oder verschiedene $C_{1-12}$-Alkylgruppen oder durch ein oder zwei gleiche oder verschiedene Halogenatome substituierten zweiwertigen $C_{6-20}$ aromatischen oder $C_{4-20}\pi$-elektronenreichen heterocyclischen Rest oder

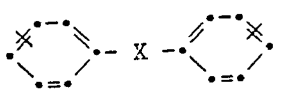

bedeutet, worin X die direkte Bindung, $—CH_2—$, —O—, —S— oder —NH— ist, sowie die entsprechenden Salze mit organischen oder anorganischen Säuren.

Aromatische oder $\pi$-elektronenreiche heterocyclische Reste $R_4$ können ein-, zwei- oder mehrkernige Systeme darstellen. Bedeutet $R_4$ einen heterocyclischen Rest, so kann dieser ein Sauerstoff- oder Schwefelatom oder eine Gruppe $—NR_5$ enthalten, worin $R_5$ Wasserstoff oder $C_{1-4}$-Alkyl bedeutet. Stellt $R_4$ ein zwei- oder mehrkerniges System dar, so können zwei oder mehr aromatische oder $\pi$-elektronenreiche heterocyclische Reste $R_4$ direkt über eine einfache Bindung, eine gesättigte oder ungesättigte Kohlenstoffkette mit 1-4 C-Atomen, über ein O- oder S-Atom oder eine NH-Gruppe miteinander verbunden sein, oder die aromatischen und/oder $\pi$-electronenreichen heterocyclischen Reste können zu einem mehrkernigen Ringsystem kondensiert sein. Aromatische oder $\pi$-elektronenreiche heterocyclische Reste $R_4$ können gegebenenfalls durch eine oder zwei gleiche oder verschiedene Halogenatome substituiert sein. Halogensubstituenten sind z. B. F-, Cl-, Br- oder J-Atome.

Stellt $R_1$ eine Alkylgruppe dar, so handelt es sich z. B. um eine Methyl-, Aethyl-, n-Propyl-, Isopropyl-, n-Butyl-, Isobutyl-, sek.-Butyl-, n-Amyl, n-Hexyl, Hept-3-yl- oder n-Octylgruppe. Stellen $R_2$ und/oder $R_5$ Alkylgruppen dar, so können diese z. B. Methyl, Aethyl, n-Propyl, Isopropyl, n-Butyl, Isobutyl oder sek.-Butyl sein. Bilden $R_1$ und $R_2$ zusammen mit dem C-Atom, an das sie gebunden sind, eine Cycloalkylenkette, so handelt es sich z. B. um einen Cyclopentylen-, Cyclohexylen-, Cycloheptylen- oder Cyclooctylenrest. $R_3$ in der Bedeutung einer Alkylgruppe ist z. B. eine Methyl-, Aethyl-, n-Propyl-, Isopropyl-, n-Butyl-, sek.-Butyl-, Amyl- oder Hexylgruppe. Beispiele von Cycloalkylgruppen $R_3$ sind die Cyclopropyl-, Cyclobutyl-, Cyclopentyl-, Cyclohexyl-, Cycloheptyl- und Cyclooctylgruppe. Bedeutet $R_3$ eine Arylgruppe, so handelt es sich z. B. um eine Phenyl- oder Naphthylgruppe. Ein- oder mehrwertige aromatische Reste $R_4$ können ein- oder mehrwertige Benzol-, Chlorbenzol-, Toluol-, Xylol-, Isopropylbenzol-, n-Octylbenzol-, Diphenyl-, Diphenyläther-, Diphenylsulfid-, Diphenylamin-, Diphenylmethan-, Diphenyläthan-, Diphenylbutan-, Triphenylmethan-, Naphthalin-, oder Phenanthrenreste sein.

Stellt $R_4$ einen $\pi$-elektronenreichen heterocyclischen Rest dar, so handelt es sich z. B. um einen Thiophen-, Methylthiophen-, Pyrrol-, Furan-, Benzofuran-, Benzthiophen- oder Indolrest.

Beispiele von Salzen von Verbindungen der Formel I sind Hydrochloride, Hydrobromide, Sulfate, Phosphate, Methansulfate, p-Toluolsulfonate, Formiate, Oxalate und Benzoate.

Bevorzugt sind Verbindungen der Formel I, worin $—ONH_2$ die oben angegebene Bedeutung hat, n 3 bis 15, $R_1$ $C_{1-6}$-Alkyl und $R_2$ $C_{1-3}$-Alkyl bedeuten, $R_3$ die oben angegebene Bedeutung hat und $R_4$ einen gegebenenfalls durch eine oder zwei gleiche oder verschiedene $C_{1-12}$-Alkylgruppen oder durch ein oder zwei gleiche oder verschiedene Halogenatome substituierten zweiwertigen $C_{6-20}$ aromatischen oder $C_{4-8}$ $\pi$-elektronenreichen heterocyclischen S-haltigen Rest oder

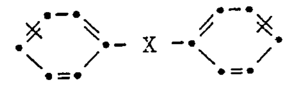

bedeutet, worin X die direkte Bindung, —$CH_2$—, —O—, —S— oder —NH—ist.

Besonders bevorzugt sind Verbindungen der Formel I, worin die Reste —$QNH_2$ die oben angegebene, vorzugsweise die gleiche Bedeutung haben, n 3 bis 15, $R_1$ $C_{1-6}$-Alkyl, $R_2$ $C_{1-3}$-Alkyl, $R_3$ Wasserstoff oder $C_{1-6}$-Alkyl und $R_4$ 1,4-Phenylen oder

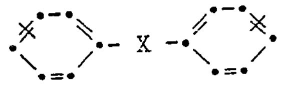

bedeuten, worin X die direkte Bindung, —$CH_2$—, —O—, —S— oder —NH— ist.

Ganz besonders bevorzugt sind Verbindungen der Formel I, worin —$QNH_2$ die oben angegebene Bedeutung hat, wobei n 3 bis 9, besonders 3, 8 oder 9, $R_1$ $C_{1-4}$-Alkyl, besonders Methyl oder Aethyl, $R_2$ Methyl oder Aethyl, vor allem Methyl, $R_3$ $C_{1-6}$-Alkyl, besonders $C_{1-4}$-Alkyl, insbesondere Methyl oder Isopropyl und $R_4$ 1,4-Phenylen oder

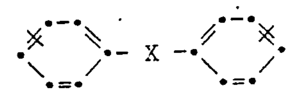

darstellen, worin X die direkte Bindung, —$CH_2$, —O— oder —NH— ist.

Bevorzugte Verbindungen der Formel I stellt auch ein Gemisch aus 2,4- und 2,5-Bis (6-amino-2-methyl-hept-2-yl)-thiophen.

Als Beispiele von Verbindungen der Formel I seien erwähnt :

2,4-Bis-(6-amino-2-methylhept-2-yl) thiophen
2,5-Bis-(6-amino-2-methylhept-2-yl) thiophen
1,4-Bis-(6-amino-2-methylhept-2-yl) benzol
Bis-1,4-(12-amino-2,13-dimethyltetradec-2-yl)-benzol
1-(12-Amino-3,13-dimethyltetradec-3-yl)-4-(12-amino-2,13-dimethyltetradec-2-yl)-benzol
4,4'-Bis-(6-amino-2-methylhept-2-yl) diphenyl
4,4'-Bis-(6-amino-2-methylhept-2-yl) diphenylmethan
4,4'-Bis-(6-amino-2-methylhept-2-yl) diphenyläther
4-(6-Amino-2-methylhept-2-yl)-4'-(12-amino-2,13-dimethyltetradec-2-yl) diphenyläther
4,4'-Bis-(6-amino-2-methylhept-2-yl) diphenylsulfid
4,4'-Bis-(6-amino-2-methylhept-2-yl)-diphenylamin
2,6-Bis-(6-amino-2-methylhept-2-yl) naphthalin
2,7-Bis-(6-amino-2-methylhept-2-yl) naphthalin
4,4'-Bis-(6-amino-2-methylhept-2-yl)-1,2-diphenyläthan
4,4'-Bis-(6-amino-2-methylhept-2-yl)-1,1-diphenyläthan
3,9-Bis-(6-amino-2-methylhept-2-yl)-phenanthren
3,10-Bis-(6-amino-2-methylhept-2-yl)-phenanthren.

Bevorzugte Verbindungen der Formel I sind :

Gemische von 2,4- und 2,5-Bis-(6-amino-2-methylhept-2-yl)-thiophen, 1,4-Bis-(6-amino-2-methylhept-2-yl)-benzol, 4,4'-Bis-(6-amino-2-methylhept-2-yl)-diphenyl, Bis-(6-amino-2-methylhept-2-yl)-diphenyläther, Bis-(6-amino-2-methylhept-2-yl)-diphenylmethan, 4,4'-Bis-(6-amino-2-methylhept-2-yl)-diphenylamin.

Gegenstand der Erfindung ist auch ein Verfahren zur Herstellung von Verbindungen der Formel I, indem man bei einer Temperatur zwischen 0 und 150 °C bevorzugt 15 und 110 °C, in Gegenwart eines Friedel-Crafts-Katalysators eine Verbindung der Formel III

$$R_4Z_2 \qquad (III),$$

worin $R_4$ die oben angegebene Bedeutung hat und Z ein ersetzbares Wasserstoffatom darstellt, mit mindestens 2 Mol eines als Alkylierungsmittel dienenden Aminoalkohols oder Aminolefins oder eines Salzes davon mit organischen oder anorganischen Säuren, die dazu befähigt sind, in der Verbindung der Formel III Z durch eine Gruppe der Formel II zu ersetzen, umsetzt.

Bevorzugt verwendet man als Alkylierungsmittel Aminoalkohole oder Salze davon mit organischen oder anorganischen Säuren, die zur Einführung eines Restes der Formel II oder zur Umwandlung in einen Rest der Formel II befähigt sind. Die Reste der Formel II, die in die Verbindung der Formel III eingeführt werden, können gleich oder verschieden sein und gewünschtenfalls auch stufenweise eingeführt werden.

Als Katalysatoren können Brönsted- oder Lewis-Säuren eingesetzt werden. Geeignete Brönsted-Säuren für diesen Zweck sind Schwefelsäure, Phosphorsäure oder Chlorwasserstoffsäure ; bevorzugte Lewis-Säuren sind Metallhalogenide, z. B. $ZnCl_2$, $SnCl_4$ oder $FeCl_3$ und vor allem $AlCl_3$.

Die Umsetzung wird zweckmässig bei Raumtemperatur und bei Atmosphärendruck sowie in Gegenwart eines inerten Lösungsmittels vorgenommen, wobei die Art des Lösungsmittels vom eingesetzten Katalysator abhängt. Für Umsetzungen unter Verwendung von Brönsted-Säuren können nichtreaktive Lösungsmitel, wie Alkohole und Wasser, als Verdünnungsmittel verwendet werden. Schwefelsäure kann z. B. zusammen mit Methanol oder Wasser verwendet werden, während Chlorwasserstoff in wässriger Lösung eingesetzt werden kann. Lewis-Säuren, wie $AlCl_3$, werden zweckmässig mit Nitromethan und chlorierten Kohlenwasserstoffen verwendet.

Nach Beendigung der Alkylierung, können die aminoalkyl-substituierten Verbindungen durch Behandlung mit wässriger Base vom Brönsted- oder Lewis-Säuren-Katalysator befreit werden. Geeignete Basen dafür sind Alkalimethallhydroxide oder Ammoniumhydroxid. Bevorzugte Basen sind Natrium- und Ammoniumhydroxid. Das Reaktionsprodukt wird mit einem [mit Wasser] nicht mischbaren organischen Lösungsmittel, wie Diäthyläther, aus der wässrigen Phase abgetrennt. Nach dem Waschen mit Wasser kann das Endprodukt durch Destillation unter vermindertem Druck erhalten werden.

Die aromatischen Reaktanden $R_4Z_2$ können in die folgenden drei Kategorien aufgeteilt werden :

1) Einkernige Aromaten oder $\pi$-elektronenreiche Heterocyclen

Diese Gruppe umfasst Benzol, alkyl- oder halogen-substituierte Benzole, monocyclische Heterocyclen und alkyl-substituierte monocyclische Heterocyclen. Beispiele solcher Verbindungen sind : Benzol, Toluol, Aethylbenzol, p-Propylbenzol, Cumol, n-Butylbenzol, sek.-Butylbenzol, tert.-Butylbenzol, n-Octylbenzol, tert.-Octylbenzol [1,1,3,3-Tetramethylbutan], o-Xylol, Chlorbenzol, Thiphen, 2-Methylthiophen, 3-Methylthiophen.

2) Verknüpfte Aromaten

Dabei sind eine oder mehrere Arylgruppen über eine Einfachbindung oder über ein oder mehrere andere Atome, wie C-Atome ($C_{1-4}$), Sauerstoff-, Schwefel- oder Stickstoffatome, direkt miteinander verbunden. Beispiele solcher Verbindungen sind : Diphenyl, Terphenyl, Diphenylmethan, 1,2-Diphenyläthan, 1,1-Diphenyläthan, Triphenylmethan, Diphenyläther, Diphenylsulfid, Diphenylamin.

3) Kondensierte Aromaten oder $\pi$-elektronenreiche Heterocyclen

Dabei handelt es sich um Verbindungen, bei denen zwei oder mehr aromatische Reste, die benachbarte C-Atome teilen, zu zwei- oder mehrkernigen Aromaten oder $\pi$-elektronenreichen Heterocyclen kondensiert sind. Das kondensierte aromatische System kann durch eine oder mehr $C_{1-4}$-Alkylgruppen oder durch Halogen substituiert sein. Beispiele solcher Verbindungen sind : Naphthalin, 1-Methylnaphthalin, 2-Methylnaphthalin, 1-Chlornaphthalin, 2-Chlornaphthalin, 1,3-Dimethylnaphthalin, 1,4-Dimethylnaphthalin, 1,5-Dimethylnaphthalin, 2,3-Dimethylnaphthalin, Isomerengemische von Dimethylnaphthalinen, Anthracen, Penanthren, Benzthiophen.

Verbindungen, die zur Einführung von Gruppen der Formel II in das Molekül des aromatischen Systems der Formel $R_4Z_2$ befähigt sind, umfassen solche der Formel IVa

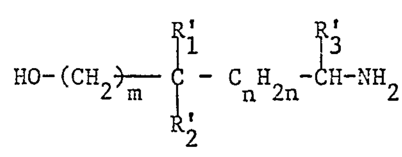

(IVa)

worin $R_3$ die oben angegebene Bedeutung hat und, wenn m = 0, n 1 bis 15, $R_1'C_{1-8}$-Alkyl, $R_2'C_{1-4}$-Alkyl oder $R_1'$ und $R_2'$ zusammen mit dem C-Atom, an das sie gebunden sind, einen $C_{5-8}$-Cycloalkylenrest darstellen, und wenn m = 1, n 1 bis 14, $R_1'$ $C_{1-7}$-Alkyl, $R_2'$ Wasserstoff oder $C_{1-3}$-Alkyl oder $R_1'$ und $R_2'$ zusammen mit dem C-Atom, an das sie gebunden sind, einen $C_{5-7}$-Cycloalkylenrest bedeuten. Wenn m in den Verbindungen der Formel IVa 1 ist, tritt eine Verschiebung der Gruppe $(CH_2)_m$ in eine der Gruppen $R_1'$, $R_2'$ oder $—C_nH_{2n}—$ ein, wodurch sich die C-Zahl dieser Gruppe um eins erhöht.

Beispiele von Verbindungen der Formel IVa sind solche, worin m = 0, n = 3 und $R_1'$ und $R_2'$ je Methyl sind ; m = 0, n = 8, $R_1'$ und $R_2'$ je Methyl und $R_3$ Isopropyl bedeuten, oder m = 1, n = 8 und $R_1'$, $R_2'$ und $R_3$ die folgende Bedeutung haben :

| $R_1'$ | $R_2'$ | $R_3$ |
|---|---|---|
| $CH_3$ | $CH_3$ | $C_2H_5$ |

(Fortsetzung)

| | | |
|---|---|---|
| $CH_3$ | $CH_3$ | $i-C_3H_7$ |
| $C_3H_7$ | $C_3H_7$ | $CH_3$ |
| $C_2H_5$ | $C_2H_5$ | $C_4H_9$ |
| 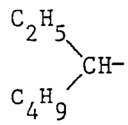 | $C_2H_5$ | $C_4H_9$ |
| $-(CH_2)_5-$ | | 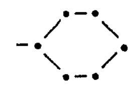 |
| $C_2H_5$ | $C_2H_5$ | $(C_2H_5)_2CH-$ |
| $CH_3$ | H | $C_6H_5$ |

Als Aminoalkohole eignen sich entsprechende, aus 11-Aminoundecanolen der Formel V

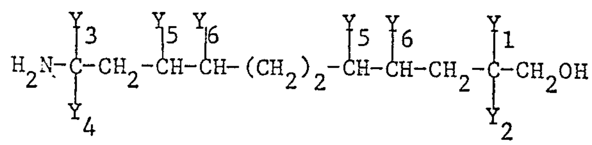

(V)

ausgewählte Verbindungen, worin $Y_1$ und $Y_3$ unabhängig voneinander Wasserstoff oder $C_{1-8}$-Alkyl, $Y_2$ und $Y_4$ unahbängig voneinander $C_{1-8}$-Alkyl oder $Y_1$ und $Y_2$ und/oder $Y_3$ und $Y_4$ zusammen mit dem C-Atom, an das sie gebunden sind, einen $C_{4-8}$ cycloaliphatischen Ring bedeuten und $Y_5$ und $Y_6$ unabhängig voneinander Wasserstoff oder $C_{1-4}$-Alkyl darstellen. Diese 11-Aminoundecanole und das Verfahren zu deren Herstellung sind in der DE-OS 2 831 299 näher beschrieben.

Besonders bevorzugt sind Heptaminol [2-Amino-6-hydroxy-6-methyl-heptan] und 11-Amino-2,2,12-trimethyl-tridecan-1-ol.

Als Beispiele von Aminoolefinen die zur Einführung einer Gruppe der Formel II in das Molekül des aromatischen Systems $R_4Z_2$ befähigt sind, seien 2-Amino-6-methyl-hept-5-en und 2-Amino-6-methyl-hept-6-en sowie die durch Dehydrierung der oben beschriebenen Aminoalkoholen erhaltenen Olefine genannt.

Die Art der Substitution des aromatischen Systems durch die Alkylaminogruppe hängt direkt vom aromatischen System selbst ab. Bei gewissen aromatischen Systemen führt die Alkylierung zu einem einzigen Isomeren, während bei anderen Isomerengemische gebildet werden.

Aromatische Verbindungen, wie Benzol und Diphenyl, ergaben 1,4- oder 4,4'-Substitution, während bei anderen Aromaten, wie Naphthalin, Diphenylmethan, Diphenyläther und Thiophen zwei oder mehr Isomere gebildet werden. Diphenylamin ergibt das 4,4'-disubstituierte Produkt.

Die erfindungsgemässen Verbindungen stellen wertvolle Zwischenprodukte zur Herstellung von Polyamiden und Kunststoffen dar.

Die neuen, aus Verbindungen der Formel I hergestellten transparenten Polyamide zeichnen sich durch verbesserte thermoplastische Verarbeitbarkeit, gute Kochwasserbeständigkeit sowie niedere Wasseraufnahme, hohe Hydrolysebeständigkeit, gute Dimensionsstabilität unter dem Einfluss von Feuchtigkeit und entsprechend verbesserte mechanische und elektrische Eigenschaften aus. Sie haben eine reduzierte spezifische Viskosität von mindestens 0,3 dl/g, bevorzugt etwa 0,5 bis etwa 2,0 dl/g und ganz besonders etwa 0,7 bis etwa 1,8 dl/g, gemessen an einer 0,5 %igen Lösung in m-Kresol bei 25 °C, und bestehen aus wiederkehrenden Strukturelementen der Formel VI

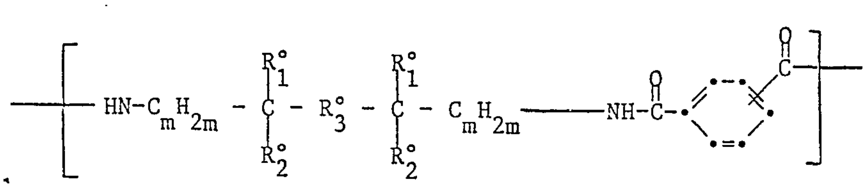

(VI)

worin die beiden m unabhängig voneinander eine ganze Zahl von 4 bis 16, die beiden $R_1$ unabhängig voneinander $C_{1-6}$-Alkyl, die beiden $R_2°$ unabhängig voneinander $C_{1-3}$-Alkyl und $R_3°$ 1,4-Phenylen oder

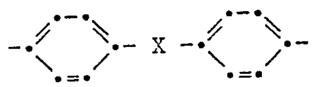

darstellen, worin X die direkte Bindung, $-CH_2-$, —O—, —S— oder —NH— ist und wobei die Carbonylgruppen in 1,3- und/oder 1,4-Stellung an den Benzolring gebunden sind.

Bevorzugt haben die beiden m dieselbe Bedeutung, die beiden $R_1°$ sind vorzugsweise gleiche Alkylgruppen und die beiden $R_2°$ sind mit Vorteil identische Alkylgruppen.

In den folgenden Beispielen bedeuten Teile und Prozente Gewichtsteile bzw. Gewichtsprozente. Die Temperaturen sind in °C angegeben.

## Beispiel 1

Zu 4,2 Teilen Thiophen und 18,2 Teilen 6-Hydroxy-6-methyl-2-heptylamin-Hydrochlorid (Heptaminol-Hydrochlorid) in 50 Teile Nitromethan gibt man bei Raumtemperatur 26,7 Teile Aluminiumchlorid in 50 Teilen Nitromethan. Nach Lagerung während zweier weiterer Tage bei Raumtemperatur wird die homogene Lösung in Wasser gegossen und solange mit Aetznatronlösung behandelt, bis die wässrige Phase alkalisch ist. Die organische Phase wird mit Diäthyläther extrahiert, mit Wasser gewaschen, verdampft und destilliert. Man erhält ein Gemisch von 2,4- und 2,5-Bis-(6-amino-2-methylhept-2-yl)-thiophen, Sdp. (0,8 mbar) 174-180°, mit der folgenden Zusammensetzung in % :

| | C | H | N |
|---|---|---|---|
| gefunden | 70,76 | 11,68 | 7,93 |
| berechnet für $C_{20}H_{38}N_8S_2$ | 70,96 | 11,31 | 8,27 |

## Beispiel 2

Zu einer Lösung von 256 Teilen 98 %iger Schwefelsäure, 48 Teilen Methanol und 7,8 Teilen Benzol werden bei Raumtemperatur unter Rühren 36,0 Teile Heptaminol-Hydrochlorid gegeben und zwar in Portionen von je 3,0 Teilen alle 6 Stunden. Nach der letzten Zugabe nach 66 Stunden wird das Reaktionsgemisch noch 18 Stunden bei Raumtemperatur gerührt und dann mit Wasser verdünnt. Die wässrige Lösung wird mit Natriumhydroxidlösung neutralisiert, und die organische Phase wird mit Diäthyläther extrahiert, mit Wasser gewaschen, verdampft und destilliert. Man erhält 18,7 Teile 1,4-Bis-(6-amino-2-methylhept-2-yl)-benzol ; Sdp. (0,8 mbar) 180-190°, mit der folgenden Zusammensetzung in % :

| | C | H | N |
|---|---|---|---|
| gefunden | 79,45 | 12,24 | 9,01 |
| berechnet für $C_{22}H_{40}N_2$ | 79,45 | 12,12 | 8,42 |

## Beispiel 3.

Nach der in Beispiel 2 beschriebenen Arbeitsweise werden 15,4 Teile Diphenyl mit 42,0 Teilen Heptaminol-Hydrochlorid umgesetzt. Die Destillation ergibt 29,0 Teile 4,4'-Bis-(6-amino-2-methylhept-2-yl)-diphenyl, Sdp. (0,3 mbar), 220-235° mit der folgenden Zusammensetzung in % :

| | C | H | N |
|---|---|---|---|
| gefunden | 81,77 | 11,04 | 6,84 |
| berechnet für $C_{28}H_{44}N_2$ | 82,29 | 10,85 | 6,85 |

## Beispiel 4

Zu 8,5 Teilen Diphenyläther und 26,7 Teilen Aluminiumchlorid in 50 Teilen Nitromethan gibt man portionenweise 18,2 Teile Heptaminol-Hydrochlorid. Nach Beendigung der Zugabe wird das Reaktionsgemisch 3 Tage bei Raumtemperatur gelagert und dann nach dem in Beispiel 1 beschriebenen Verfahren aufgearbeitet. Die Destillation ergibt 2,1 Teile 4-(6-Amino-2-methylhept-2-yl)-diphenyläther, Sdp. (0,4 mbar) 170-180°, gefolgt von 14,2 Teilen Bis-(6-amino-2-methylhept-2-yl)-diphenyläther, Sdp. (0,7 mbar 240-270°. Gemäss Analyse besteht diese zweite Fraktion aus 70 % des 4,4'-Isomeren. Die Zusammensetzungen der ersten und zweiten Fraktionen sind wie folgt (in %) :

| | C | H | N |
|---|---|---|---|
| gefunden | 80,38 | 9,74 | 5,28 |
| berechnet für $C_{20}H_{27}NO$ | 80,76 | 9,15 | 4,71 |

| | N | H | N |
|---|---|---|---|
| gefunden | 79,31 | 10,61 | 6,72 |
| berechnet für $C_{28}H_{44}N_2O$ | 79,19 | 10,44 | 6,60 |

Beispiel 5

16,8 Teile Diphenylmethan werden nach dem in Beispiel 2 beschriebenen Verfahren mit 36,4 Teilen Heptaminol-Hydrochlorid umgesetzt. Die Destillation ergibt 22,4 Teile Bis-(6-amino-2-methylhept-2-yl)-diphenylmethan, Sdp. (0,4 mbar) 230-244°. Gemäss Analyse besteht diese Fraktion aus 73 % des 4,4'-Isomeren mit der folgenden Zusammensetzung in % :

| | C | H | N |
|---|---|---|---|
| gefunden | 82,71 | 11,31 | 6,55 |
| berechnet für $C_{29}H_{46}N_2$ | 82,40 | 10,97 | 6,63 |

Beispiel 6

16,9 Teile Diphenylamin, 20,4 Teile 36 %ige wässrige Chlorwasserstoffsäure, 6,8 Teile wasserfreies Zinkchlorid, 36,4 Teile Heptaminol-Hydrochlorid und 20 Teile Wasser werden 4 Tage bei Rückfluss gerührt. Nach dem Abkühlen wird das Reaktionsgemisch mit 50 Teilen Natriumhydroxid in 100 Teilen Wasser behandelt, und die organische Phase wird mit Diäthyläther extrahiert, mit Wasser gewaschen, verdampft und destilliert. Nach dem Entfernen von 8,0 Teilen einer ersten Fraktion, Sdp. (0,33 mbar) 146-238°, erhält man 29,6 Teile 4,4'-Bis-(6-amino-2-methylhept-2-yl)-diphenylamin, Sdp. (0,3 mbar 254-258° (70 % der Theorie), mit der folgenden Zusammensetzung in % :

| | C | H | N |
|---|---|---|---|
| gefunden | 79,59 | 10,93 | 9,84 |
| berechnet für $C_{28}H_{45}N_3$ | 79,37 | 10,70 | 9,91 |

Beispiel 7

In einem mit Rührwerk, Rückflusskühler und Tropfrichter versehenen Kolben werden 2,556 g (0,0154 Mol) Terephthalsäure in 100 ml 50 %igem Aethanol aufgeschlämmt und auf Rückflusstemperatur erhitzt. In die siedende Mischung werden aus dem Tropftrichter 5,137 g (0,0154 Mol) 1,4-Bis-(6-amino-2-methylhept-2-yl)-benzol eingetropft. Es entsteht eine klare Lösung, aus welcher sich nach wenigen Minuten das gebildete Salz ausscheidet. Das Salz wird abfiltriert und bei 80° im Vakuum getrocknet. Ausbeute : 6,5 g (85 % der Theorie). Das Salz wird dann in ein Bombenrohr, das mit Schraudbeckel und eingebautem Ueberdruckventil versehen ist, gegeben. Nachdem man die Luft im Bombenrohr vollständig durch Stickstoff verdrängt hat, verschliesst man das Bombenrohr und taucht es in ein Salzbad von 270° ein. Nach kurzer Zeit ist eine klare Schmelze entstanden. Nach zwei Stunden wird die Reaktion abgebrochen, indem man das Rohr aus dem Salzbad entfernt und den Ueberdruck durch Oeffnen des Ventils ablässt. Das erstarrte Vorkondensat wird aus dem Bombenrohr entfernt und in eine Kondensationsgefäss übergeführt. Unter strengem Ausschluss von Luft und dauerndem Durchleiten von Stickstoff bei einer Temperatur von 280° wird die gebildete Schmelze polykondensiert, wobei das entstandene Wasser durch den Stickstoffstrom laufen entfernt wird. Nach 5 Stunden wird die Polykondensation abgebrochen. Beim Abkühlen erstarrt die Schmelze zu einer glasklaren farblosen Masse.

2-3 g des so erhaltenen Polyamids werden mittels einer beheizbaren hydraulischen Presse zu einer Folie von ca. 0,3 bis 0,5 mm Dicke verpresst. Zur Bestimmung der Wasseraufnahme wird die Folie bei Raumtemperatur einer relativen Luftfeuchtigkeit von 65 % ausgesetzt, bis keine Gewichtszunahme mehr festgestellt werden kann. Die reduzierte Lösungsviskosität des erhaltenen Polyamids gemessen an einer 0,5 %igen Lösung in m-Kresol bei 25° beträgt 1,13 dl/g. Die Glasumwandlungstemperatur Tg wird im Differentialkalorimeter (DSC) bestimmt und beträgt 135°.

**Patentansprüche**

1. Verbindungen der Formel I

$$R_4(QNH_2)_2 \qquad (I),$$

worin die Reste $—QNH_2$ gleiche oder verschiedene Gruppen der Formel II

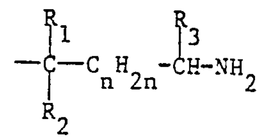

bedeuten, worin n eine ganze Zahl von 1-15, $R_1$ $C_{1\text{-}8}$-Alkyl, $R_2$ $C_{1\text{-}4}$-Alkyl oder $R_1$ und $R_2$ zusammen mit dem C-Atom, an das sie gebunden sind, eine $C_{5\text{-}8}$-Cycloalkylengruppe und $R_3$ Wasserstoff, $C_{1\text{-}6}$-Alkyl, $C_{3\text{-}8}$-Cycloalkyl oder $C_{6\text{-}10}$-Aryl bedeuten, mit der Massgabe, dass wenn $R_4$ = 1,4-Phenylen, n = 3 und $R_1 = R_2$ = Methyl sind, $R_3$ verschieden von Wasserstoff ist, und $R_4$ einen gegebenenfalls durch eine oder zwei gleiche oder verschiedene $C_{1\text{-}12}$-Alkylgruppen oder durch ein oder zwei gleiche oder verschiedene Halogenatome substituierten zweiwertigen $C_{6\text{-}20}$ aromatischen oder $C_{4\text{-}20}$ $\pi$-elektronenreichen heterocyclischen Rest oder

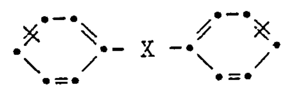

bedeutet, worin X die direkte Bindung, —$CH_2$—, —O—, —S— oder —NH— ist, sowie die entsprechenden Salze mit organischen oder anorganischen Säuren.

2. Verbindungen der Formel I nach Anspruch 1, worin —$QNH_2$ die in Anspruch 1 angegebene Bedeutung hat, n 3 bis 15, $R_1$ $C_{1\text{-}6}$-Alkyl und $R_2$ $C_{1\text{-}3}$-Alkyl bedeuten, $R_3$ die im Anspruch 1 angegebene Bedeutung hat und $R_4$ einen gegebenenfalls durch eine oder zwei gleiche oder verschiedene $C_{1\text{-}12}$-Alkylgruppen oder durch ein oder zwei gleiche oder verschiedene Halogenatome substituierten zweiwertigen $C_{6\text{-}20}$ aromatischen oder $C_{4\text{-}8}$ $\pi$-elektronenreichen heterocyclischen S-haltigen heterocyclischen Rest oder

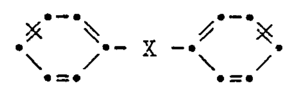

bedeutet, worin X die direkte Bindung, —$CH_2$—, —O—, —S— oder —NH— ist.

3. Verbindungen der Formel I nach Anspruch 1, worin die Reste —$QNH_2$ die im Anspruch 1 angegebene Bedeutung haben, n 3 bis 15, $R_1$ $C_{1\text{-}6}$-Alkyl, $R_2$ $C_{1\text{-}3}$-Alkyl, $R_3$ Wasserstoff oder $C_{1\text{-}6}$-Alkyl, und $R_4$ 1,4-Phenylen oder

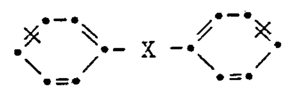

bedeuten, worin X die direkte Bindung, —$CH_2$—, —O—, —S— oder —NH— ist.

4. Verbindungen der Formel I nach Anspruch 3, worin die —$QNH_2$ gleiche Reste darstellen.

5. Verbindungen der Formel I nach Anspruch 1, worin —$QNH_2$ die im Anspruch 1 angegebene Bedeutung hat, n 3 bis 9, $R_1$ $C_{1\text{-}4}$-Alkyl, $R_2$ Methyl oder Aethyl, $R_3$ $C_{1\text{-}6}$-Alkyl und $R_4$ 1,4-Phenylen oder

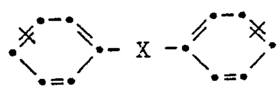

darstellen, worin X die direkte Bindung, —$CH_2$, —O— oder —NH— ist.

6. Verbindungen der Formel I nach Anspruch 5, worin n 3, 8 oder 9 ist, $R_1$ Methyl oder Aethyl, $R_2$ Methyl und $R_3$ $C_{1\text{-}4}$-Alkyl bedeuten.

7. Verbindungen der Formel I nach Anspruch 6, worin $R_3$ Methyl oder Isopropyl ist.

8. Gemisch aus 2,4- und 2,5-Bis-(6-amino-2-methylhept-2-yl)-thiophen nach Anspruch 1.

9. Verfahren zur Herstellung von Verbindungen der Formel I oder Salzen davon mit organischen oder anorganischen Säuren nach Anspruch 1, dadurch gekennzeichnet, dass man bei einer Temperatur zwischen 0 und 150 °C und in Gegenwart eines Friedel-Crafts-Katalysators eine Verbindung der Formel III

$$R_4(Z)_2 \qquad (III),$$

worin $R_4$ die im Anspruch 1 angegebene Bedeutung hat und Z ein ersetzbares Wasserstoffatom darstellt, mit mindestens 2 Mol eines als Alkylierungsmittel dienenden Aminoalkohols oder Aminolefins oder eines Salzes davon mit organischen oder anorganischen Säuren umsetzt, die befähigt sind, in der Verbindung der Formel III Z durch eine Gruppe der Formel II zu ersetzen.

10. Verfahren nach Anspruch 9, worin als Alkylierungsmittel Aminoalkohole oder Salze davon mit einer organischen oder anorganischen Säure verwendet werden.

11. Verfahren nach Anspruch 9, worin der Katalysator eine Brönsted-Säure oder eine Lewis-Säure ist.

12. Verfahren nach Anspruch 9, worin die Brönsted-Säure Schwefelsäure, Phosphorsäure oder Chlorwasserstoffsäure ist.

13. Verfahren nach Anspruch 9, worin die Lewis-Säure ein Metallhalogenid, vorzugsweise $AlCl_3$, ist.

14. Verfahren nach Anspruch 9, dadurch gekennzeichnet, dass die Umsetzung bei Rautemperatur und Atmosphärendruck und in Gegenwart eines inerten Lösungsmittels durchgeführt wird.

**Claims**

1. A compound of the formula I

$$R_4(QNH_2)_2 \quad (I),$$

wherein the residues $—QNH_2$ are identical or different groups of the formula

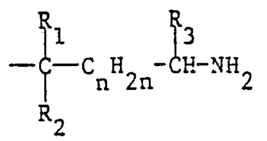

(II)

wherein n is an integer from 1 to 15, $R_1$ is $C_1$-$C_8$ alkyl, $R_2$ is $C_1$-$C_4$ alkyl or $R_1$ and $R_2$, together with the carbon atom to which they are attached, form a $C_5$-$C_8$ cycloalkylene group, and $R_3$ is hydrogen, $C_1$-$C_6$ alkyl, $C_3$-$C_8$ cycloalkyl or $C_6$-$C_{10}$ aryl, with the proviso that, if $R_4$ is 1,4-phenylene, n is 3 and $R_1$ and $R_2$ are methyl, $R_3$ is different from hydrogen, and $R_4$ is a divalent $C_6$-$C_{20}$ aromatic or $C_4$-$C_{20}$ $\pi$-excessive heterocyclic residue which is unsubstituted or substituted by one or two identical or different $C_1$-$C_{12}$ alkyl groups or by one or two identical or different halogen atoms, or is

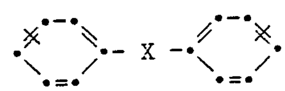

wherein X is a direct bond, $—CH_2—$, —O—, —S— or —NH— ; as well as the corresponding salts of organic or inorganic acids.

2. A coumpound of formula I according to claim 1, wherein the residue $—QNH_2$ is as defined in claim 1, n is 3 to 15, $R_1$ is $C_1$-$C_6$ alkyl, $R_2$ is $C_1$-$C_3$ alkyl, $R_3$ is as defined in claim 1 and $R_4$ is a divalent $C_6$-$C_{20}$ aromatic or $C_4$-$C_8$$\pi$-excessive heterocyclic residue containing sulfur, which is unsubstituted or substituted by one or two identical or different $C_1$-$C_{12}$ alkyl groups or by one or two identical or different halogen atoms, or is

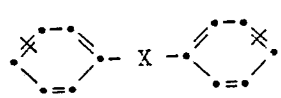

wherein X is a direct bond, $—CH_2—$, —O—, —S— or —NH—.

3. A compound of formula I according to claim 1, wherein the residues $—QNH_2$ are as defined in claim 1, n is 3 to 15, $R_1$ is $C_1$-$C_6$ alkyl, $R_2$ is $C_1$-$C_3$ alkyl, $R_3$ is hydrogen or $C_1$-$C_6$ alkyl und $R_4$ is 1,4-phenylene or

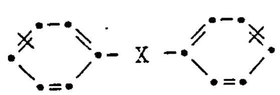

wherein X is a direct bond, $—CH_2—$, —O—, —S— or —NH—.

4. A compound of formula I according to claim 3, wherein the $—ONH_2$ residues are identical.

5. A compound of the formula I according to claim 1, wherein $—ONH_2$ is as defined in claim 1, n is 3 to 9, $R_1$ is $C_1$-$C_4$ alkyl, $R_2$ is methyl or ethyl, $R_3$ is $C_1$-$C_6$ alkyl and $R_4$ is 1,4-phenylene or is

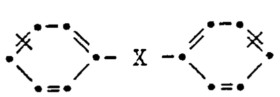

wherein X is a direct bond, —$CH_2$—, —O— or —NH—.

6. A compound of formula I according to claim 5, wherein n is 3, 8 or 9, $R_1$ is methyl or ethyl, $R_2$ is methyl and $R_3$ is $C_1$-$C_4$ alkyl.

7. A compound of formula I according to claim 6, wherein $R_3$ is methyl or isopropyl.

8. A mixture of compounds according to claim 1 consisting of 2,4- and 2,5-bis-(6-amino-2-methylhept-2-yl) thiophene.

9. A process for the preparation of a compound of formula I, or a salt thereof with an organic acid, according to claim 1, which comprises reacting, at a temperature in the range from 0° to 150 °C, in the presence of a Friedel-Crafts catalyst, a compound of the formula III

$$R_4(Z)_2 \quad \text{(III)}$$

wherein $R_4$ is as defined in claim 1 and Z is a replaceable hydrogen atom, with at least 2 moles of an alkylating agent which is an aminoalcohol or amino-olefin or of a salt thereof with an organic acid capable of replacing Z in the compound of formula III by a group of the formula II.

10. A process according to claim 9, wherein the alkylating agent is an amino-alcohol or a salt thereof with an organic or inorganic acid.

11. A process according to claim 9, wherein the catalyst is a Bronsted acid or a Lewis acid.

12. A process according to claim 9, wherein the Bronsted acid is sulfuric acid, phosphoric acid or hydrochloric acid.

13. A process according to claim 9, wherein the Lewis acid is a metal halide, preferably $AlCl_3$.

14. A process according to claim 9, wherein the reaction is carried out at room temperature and under atmospheric pressure, in the presence of an inert solvent.

**Revendications**

1. Composés répondant à la formule I

$$R_4(QNH_2)_2 \quad \text{(I)}$$

dans laquelle les radicaux —$QNH_2$ représentent des radicaux, identiques ou différents, répondant à la formule II

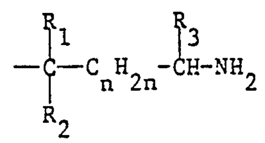

(II)

dans laquelle n représente un nombre entier de 1 à 15, $R_1$ représente un alkyle en $C_1$-$C_8$, $R_2$ représente un alkyle en $C_1$-$C_4$ ou encore $R_1$ et $R_2$ forment ensemble, et avec l'atome de carbone auxquels ils sont liés, un radical cycloalkylène en $C_5$-$C_8$, et $R_3$ représente l'hydrogène, un alkyle en $C_1$-$C_6$, un cycloalkyle en $C_3$-$C_8$ ou un aryle en $C_6$-$C_{10}$, avec la condition que $R_3$ ne peut pas représenter un atome d'hydrogène lorsque $R_4$ est un radical phénylène-1,4, que n est égal à 3 et que $R_1$ et $R_2$ représentent chacun un méthyle, et $R_4$ représente un radical aromatique bivalent en $C_6$-$C_{20}$ ou un radical hétérocyclique bivalent en $C_4$-$C_{20}$ riche en électrons $\pi$ ou un radical de formule :

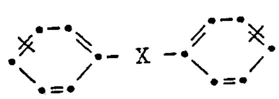

(dans lequel X représente une liaison directe ou un pont —$CH_2$—, —O—, —S— ou —NH), chacun de ces radicaux pouvant porter un ou deux alkyles en $C_1$-$C_{12}$, identiques ou différents, ou un ou deux atomes d'halogène, identiques ou différents, ainsi que les sels que forment ces composés avec des acides minéraux ou organiques.

2. Composés de formule I selon la revendication 1 dans lesquels —$QNH_2$ a la signification donnée à la renvendication 1, n désignant un nombre de 3 à 15, $R_1$ un alkyle en $C_1$-$C_6$, $R_2$ un alkyle en $C_1$-$C_3$ et $R_3$ ayant la signification donnée à la revendication 1, et $R_4$ représente un radical aromatique bivalent en $C_6$-$C_{20}$ ou un radical hétérocyclique sulfuré bivalent en $C_4$-$C_8$, riche en électrons $\pi$, ou un radical de formule :

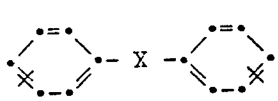

(dans lequel X représente une liaison directe ou un radical —$CH_2$—, —O—, —S— ou —NH—), chacun de ces radicaux cités pour $R_4$ pouvant porter un ou deux radicaux alkyles en $C_1$-$C_{12}$, identiques ou différents ou un ou deux atomes d'halogènes, identiques ou différents.

3. Composés de formule I selon la revendication I dans lesquels les radicaux —$QNH_2$ ont la signification donnée à la revendication 1, n désignant un nombre de 3 à 15, $R_1$ un alkyle en $C_1$-$C_6$, $R_2$ un alkyle en $C_1$-$C_3$ et $R_3$ l'hydrogène ou n alkyle en $C_1$-$C_6$, et $R_4$ représente un radical phénylène-1,4 ou un radical de formule :

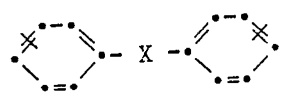

dans laquelle X représente une liaison directe ou un radical —$CH_2$—, —O—, —S— ou —NH—.

4. Composés de formule I selon la revendication 3 dans lesquels les radicaux —$QNH_2$ sont identiques l'un à l'autre.

5. Composés de formule I selon la revendication 1 dans lesquels —$QNH_2$ a la signification donnée à la revendication 1, n désignant un nombre de 3 à 9, $R_1$ un alkyle en $C_1$-$C_4$, $R_2$ un méthyle ou un éthyle et $R_3$ un alkyle en $C_1$-$C_6$, et $R_4$ représente un radical phénylène-1,4 ou un radical répondant à la formule :

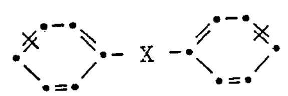

dans laquelle X représente une liaison directe ou un radical —$CH_2$—, —O— ou —NH—.

6. Composés de formule I selon la revendication 5 dans lesquels n est égal à 3, à 8 ou à 9, $R_1$ représente un méthyle ou un éthyle, $R_2$ un méthyle et $R_3$ un alkyle en $C_1$-$C_4$.

7. Composés de formule I selon la revendication 6 dans lesquels $R_3$ représente un méthyle ou un isopropyle.

8. Mélange de bis-(diméthyl-1,1 amino-5 hexyl)-2,4 et -2,5 thiophènes selon la revendication 1.

9. Procédé de préparation de composés de formule I, ou de sels formés par ceux-ci avec des acides minéraux ou organiques, selon la revendication 1, procédé caractérisé en ce qu'on fait réagir, à une température de 0 à 150 °C et en présence d'un catalyseur de Friedel et Crafts, un composé répondant à la formule III :

$$R_4(Z)_2 \quad \text{(III)}$$

dans laquelle $R_4$ a la signification donnée à la revendication 1 et Z représente un atome d'hydrogène mobile, avec au moins 2 mol d'un aminoalcool ou d'une amino-oléfine jouant le rôle d'un agent d'alkylation, ou d'un sel d'un tel composé aminé avec un acide minéral ou organique, ce composé utilisé comme agent d'alkylation, ou d'un sel d'un tel composé aminé avec un acide minéral ou organique, ce composé utilisé comme agent d'alkylation devant être capable de remplacer Z par un radical de formule II dans le composé de formule III.

10. Procédé selon la revendication 9, caractérisé en ce qu'on utilise, comme agents d'alkylation, des aminoalcools ou des sels dérivant d'aminoalcools et d'acides minéraux ou organiques.

11. Procédé selon la revendication 9, caractérisé en ce que le catalyseur est un acide de Brönsted ou un acide de Lewis.

12. Procédé selon la revendication 9, caractérisé en ce que l'acide de Brönsted est l'acide sulfurique, l'acide phosphorique ou l'acide chlorhydrique.

13. Procédé selon la revendication 9, caractérisé en ce que l'acide de Lewis est un halogénure de métal, de préférence $AlCl_3$.

14. Procédé selon la revendication 9, caractérisé en ce que la réaction est effectuée à la température ambiante, sous la pression atmosphérique et en présence d'un solvant inerte.